# EUROPEAN PATENT APPLICATION

(11) **EP 1 258 426 A2**
(43) Date of publication of application: **20.11.2002**
(21) Application number: 01127623.5
(22) Date of filing: 20.11.2001
(51) Int. Cl.: B63C 11/12, A63B 33/00

(54) **Head strap provided with ear flaps for underwater masks, particularly for swimmers of skin-divers**

(30) Priority: 16.05.2001 IT GE010046
(71) Applicant: HTM SPORT S.p.A., 16035 Rapallo (Genova) (IT)
(72) Inventor: Garofalo, Giovanni, 16035 Rapallo, Province of Genova (IT)
(74) Representative: Porsia, Attilio, Dr.

(57) **Abstract**

The head strap (4) to be applied to the belt (3) of underwater masks (1) comprises two side elements or ear flaps (5) suitable to cover completely the ear lobes (6) of the user of the mask.

## Description

The present invention has for its object the head straps or bands for underwater masks, particularly the head straps for the swimmers who practise the exploration of the sea by swimming on the surface or for the divers who make relatively non-deep immersions, like the skin-divers.

It is well-known in fact that the contact of too cold water with the ears and the quick exchange of this water in contact with the ears that occurs both during the swimming and the immersions causes an excessive cooling of the ears, that could be also cause of troubles for a person who, for example, has suffered otitis or the like. This problem can be even more serious during the immersions also at relatively moderate depths, since it is very easy to meet, few meters under the surface of the water, cold streams.

It is therefore the object of the present invention to provide a head strap or band provided with ear flaps, so as to prevent a quick exchange of the water contained between the ear flap and the internal portion of the same ear, thus avoiding the occurrence of the above mentioned troubles, due to an excessive cooling of the ears.

Advantageously, such head strap is realised in neoprene foam, or similar material. It presents in addition to the mentioned advantage, also the further advantage of a better distribution of the belt mask tension upon the nape, preventing said belt to slide upwardly or downwardly.

Further features and advantages of the head strap according to the invention will be better understood in the course of the following description of a preferred embodiment of same, made with reference to the attached drawings, in which:

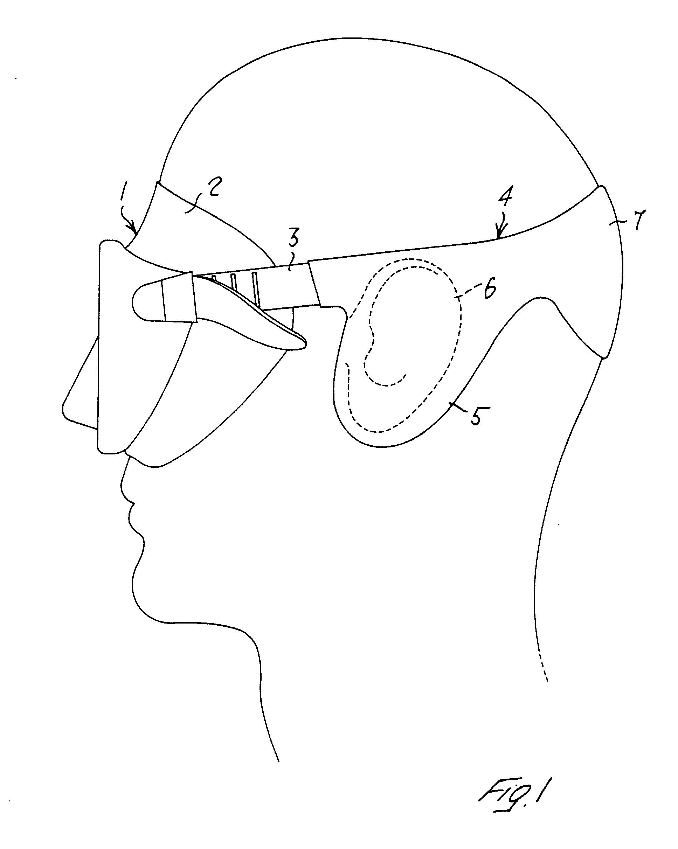

Figure 1 is a profile view of the head of one swimmer wearing a mask for underwater swimming, provided with the head strap with ear-flap according to the invention, and
Figure 2 is a plan view of the head strap according to the invention.

With reference to the drawings, with reference numeral 1 is indicated a normal mask for underwater swimming, comprising in an usual manner, a facial element 2 provided with support belt 3, passing around the nape of the neck of the user.

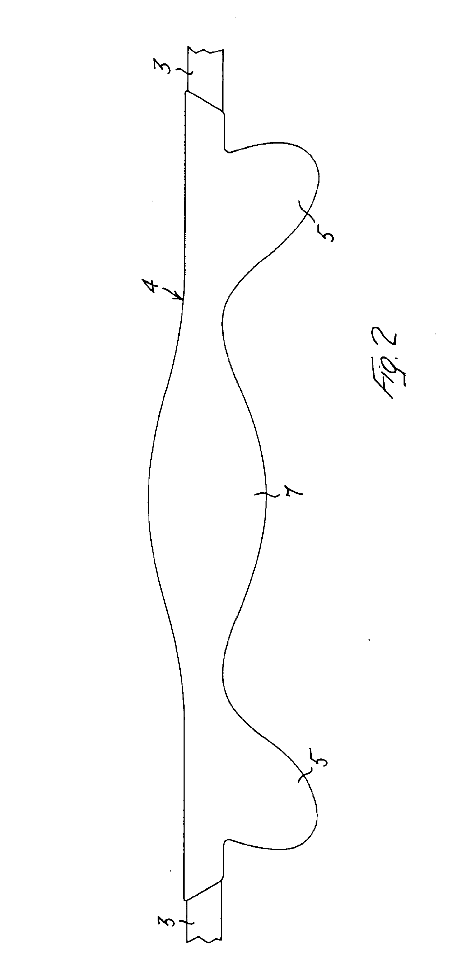

Astride the belt 3 is applied the head strap 4. Such head strap is made preferably of expanded neoprene, even if it can be made of any other material suitable for the purpose. As it is best shown in Figure 2, such head strap presents two expansions or lobes 5 at the height of ears of the user, having such dimensions as to cover completely the auricles of the ears 6 of the user. Moreover, such head strap is preferably, although not necessarily, provided, in the zone of the nape of the neck of the user, with a lozenge-shaped enlarged portion 7, suitable to be applied in a zone relatively wide of the nape of the neck of the user.

Advantageously, such head strap is obtained by welding the edges of two symmetrical sections 4, so as to provide between the two sections 4 a passage for the belt 3, or it can be realised in a manner to be applied to the belt 3 in any other manner suitable for the purpose.

It will be clear that, thanks to the presence of the ear guard flap elements 5, the water contained between the ear 6 and the ear flap 5 will not undergo to rapid exchanges, so that in this zone the water will always be at a temperature almost equal to the body temperature of the diver, so as to avoid the problems connected to the quick exchange of the water in these zones.

Moreover, thanks to the presence of the element 7 having a surface relatively wide applied against the nape of the user, the head strap according to the invention presents also the advantage of a better distribution of the tension of the mask belt upon the nape, preventing the sliding upwardly or downwardly of the belt.

Naturally the head strap according to the invention it is not limited to the illustrated and described embodiment, but includes all those variations and modifications forming part of the inventive concept. Thus, by way of example, although as it has been described a head strap constituted by a monolithic element comprising two ear flaps, it is understood that the two ear flaps can be realised in the form of single elements to be applied to the belt of an underwater mask.

## Claims

1. Head strap or band to be applied to the belt of underwater masks (1) **characterised by** the fact that it comprises two side elements or ear flaps (5) suitable to cover completely the ear lobes (6) of the user of the mask.

2. Head strap according to claim 1, **characterised by** the fact that it is made of soft insulating material, such as, for example, neoprene foam.

3. Head strap according to claim 1, **characterised by** the fact that the two ear flaps are connected between them by means of an element (7) forming a support expansion onto the nape of the user.

4. Head strap according to claim 1, **characterised by** the fact that it is constructed of a single piece of double-layer neoprene foam, a passage for the belt (3) of the underwater mask being obtained between the two layers of the head strap.
